# EUROPÄISCHE PATENTSCHRIFT

(11) **EP 1 091 700 B1**
(45) Veröffentlichungstag und Bekanntmachung des Hinweises auf die Patenterteilung: **27.04.2005**
(21) Anmeldenummer: 99931228.3
(22) Anmeldetag: 01.07.1999
(51) Int. Cl.: A61B 17/72

(54) **KNOCHENDISTRAKTIONSVORRICHTUNG**
BONE DISTRACTION DEVICE
DISPOSITIF D'ECARTEMENT POUR OS

(30) Priorität: 02.07.1998 DE 19829523
(43) Veröffentlichungstag der Anmeldung: 18.04.2001
(73) Patentinhaber: Wittenstein AG, 97999 Igersheim (DE)
(72) Erfinder: Butsch, Michael, D-88718 Daisendorf (DE)
(74) Vertreter: Weiss, Peter, Dr.
(86) Internationale Anmeldenummer: PCT/EP1999/004540
(87) Internationale Veröffentlichungsnummer: WO 2000/001315

(56) Entgegenhaltungen:
- EP-A- 0 919 717
- FR-A- 2 726 460
- NL-C- 1 004 873
- US-A- 5 156 605
- US-A- 5 415 660

## Beschreibung

Die vorliegende Erfindung betrifft eine Distraktionsvorrichtung zum Auseinanderbewegen eines einoder zweiteiligen, ggf. getrennten Knochens zur Verlängerung oder Überbrückung eines Knochenspaltes, umfassend einen ggf. in einen Markraum eines Knochens einführbaren Marknagel, welcher wenigstens ein erstes Element und ein zweites Element aufweist, welche axial gegeneinander bewegbar sind.

Derartige Vorrichtungen sind in vielfältigster Form und Ausführung auf dem Markt bekannt und gebräuchlich. Ziel dieser Distraktion ist eine körpereigene Nachbildung von Knochenmaterial. Verwiesen wird bspw. auf die EP 0 346 247 B1, in welcher ein intrakorporaler Knochenmarksnagel aufgezeigt ist, welcher zwei Teile eines Knochens auf mechanische Weise bewegt. Nachteilig daran ist, dass eine Nachstellung und ein Spannen nicht ohne weiteres möglich ist.

Ferner ist aus der DE 39 21 972 C2 ein Marknagel aufgezeigt, welcher ebenfalls auf mechanische Weise verlängerbar ist.

Die US 5,415,660 beschreibt eine Distraktionsvorrichtung bei welcher eine Schubstange gegenüber einem Gehäuse mittels einer Spanneinrichtung bewegbar ist, indem ein druckbetriebener Aktuator aus Formgedächtnismaterial die Spanneinrichtung mit Druck beaufschlagt. Zum Zurückbewegen der Spanneinrichtung sind Federelemente vorgehsehen.

Die FR 2 726 460 offenbart eine Distraktionseinrichtung, bei welcher ein erstes Element gegenüber dem zweiten Element bewegbar ist, indem zwischen zwei Rastelementen ein druckbetätigter Aktuator das eine Rastelement gegenüber dem anderen bei Druckbeaufschlagung bewegt. Zum Zurückziehen und Zusammenziehen ist ein zugbelastetes Federelement vorgesehen.

Nachteilig bei den herkömmlichen und im Stand der Technik aufgezeigten Distrakionsvorrichtungen ist, dass diese meist zu gross ausgebildet sind und einen schlechten Wirkungsgrad besitzen. Sie können nur begrenzte Distraktionskräfte auf den Knochen übertragen.

Meist sind sie komplex aufgebaut, schwierig zu bedienen und insbesondere häufig durch operative Eingriffe nachzustellen, was unerwünscht ist.

Ferner sind derartige im Stand der Technik bekannte Distraktionsvorrichtungen selten für sehr kleine Knochen geeignet, da ihre Grösse durch komplexe Bauteile begrenzt ist.

Der vorliegenden Erfindung liegt die Aufgabe zugrunde, eine Distraktionsvorrichtung der o. g. Art zu schaffen, mit geeignet, da ihre Grösse durch komplexe Bauteile begrenzt ist.

Der vorliegenden Erfindung liegt die Aufgabe zugrunde, eine Distraktionsvorrichtung der o. g. Art zu schaffen, mit welcher hohe Distraktionskräfte steuerbar auf den Knochen ausgeübt werden und welche in beliebigen Grössen für alle verlängerbaren Knochen, auch sehr kleine Knochen, einsetzbar ist. Dabei sollen Distraktionsstrecken sowie Distraktionskräfte frei wählbar sein.

Zur Lösung dieser Aufgabe führt, dass zum axialen Auseinanderbewegen des ersten Elementes gegenüber dem zweiten Element ein Halteelement vorgesehen ist, welches gegenüber dem ersten Element abgestützt ist und einends zwischen dem Halteelement und dem zweiten Element einerseits eine Arbeitseinrichtung bestehend aus zumindest einem Draht oder einem Rohr- oder rohrartigen Element aus einer Formgedächtnis-Legierung und andernends zwischen Halteelement und dem zweiten Element andererseits eine Spanneinrichtung vorgesehen ist, wobei eine Distraktion des ersten Elementes gegenüber dem zweiten Element durch Verkürzen der Arbeitseinrichtung erfolgt und ein Schubelement des zweiten Elementes gegenüber dem an dem ersten Element fixierten Halteelement in Distraktions-Richtung X bewegbar ist.

Bei der vorliegenden Erfindung sind vorzugsweise ineinander eingesetzte oder aneinander geführte Elemente vorgesehen, die verschiebbar angeordnet sind. Endseits sind die Elemente mit den jeweilgen einzelnen Knochenteilen fest verbunden.

Vorzugsweise ist das stehende Element mit einem Schubelement versehen, auf welchem sich das Halteelement axial hin und her bewegt. Das Halteelement stützt sich an dem äusseren Element mittels einer Sperreinrichtung ab. Hier können unterschiedlichste Varianten von Sperreinrichtungen gewählt sein. Das Halteelement ist über eine Arbeitseinrichtung mit dem Schubelement endseits verbunden.

Die Arbeitseinrichtung, welche vorzugsweise aus definiert in Achsrichtung gewickeltem Draht besteht, kann bei Verwendung sogenannter Formgedächtnislegierungen bei Erwärmen die Länge wesentlich ändern. Der in Achsrichtung gewickelte Draht zieht sich um mehrere Prozent der Gesamtlänge zusammen. Durch die wechselseitige Abstützung des Halteelementes an den beiden Elementen wird das Ausfahren des einen und/oder des anderen Elementes erzwungen.

Anschliessend wird die Arbeitseinrichtung durch Unterbrechung einer Stromzufuhr abgekühlt, der Draht kann sich entspannen. Eine Spanneinrichtung, welche mit dem verschiebbaren bzw. bewegbaren Element und dem Halteelement verbunden ist, bewegt das Halteelement entgegen einer Richtung zurück.

Bevorzugt wird ebenfalls als Spanneinrichtung ein Drahtelement verwendet, welches mit Strom beaufschlagt wird. Dadurch wird der Draht erwährmt und verkürzt sich, da er aus Memory-Metall hergestellt ist. Das innenliegende Element stützt sich gegenüber dem äusseren Element über eine Sperreinrichtung ab, so dass das Halteelement in Richtung bewegt werden kann. Der Vorgang wiederholt sich.

Durch die Anzahl der Drähte, welche in Arbeits- bzw. Spanneinrichtung eingesetzt sind sowie über die entsprechenden Abstände von einer Aufnahme des Schubelementes zum Halteelement bzw. vom Halteelement zum inneren Element lässt sich exakt Einfluss auf die geforderte Distraktionsstrecke und die Distraktionskraft nehmen. Anstelle des Spannelementes kann auch je nach Anordnung ein Zug- oder Druckfederelement eingesetzt werden.

Die Drähte die zur Erwärmung des Memory-Metalldrahtes dienen sind an Energieübertrager angeschlossen, welche bspw. unter der Haut angeordnet sind. Auf diese Weise kann induktiv berührungslos ohne operativen Aufwand die Distraktionseinrichtung über einen langen Zeitraum hinweg betätigt werden.

Insgesamt ist mit der vorliegenden Erfindung eine Distraktionseinrichtung geschaffen, welche auf jeden beliebigen Knochen, der verlängert werden soll, in Form, Grösse und technischer Ausführung angepasst werden kann. Es kann über bspw. die Anzahl der Drähte, über die Länge der Drähte exakt Einfluss auf das Distraktionsverhalten genommen werden. Diese Distraktionsvorrichtung ist auch für sehr hohe Distraktionskräfte bei kleinster möglicher Einbaugrösse geeignet.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 einen zumindest teilweisen dargestellten Querschnitt durch eine Distraktionsvorrichtung mit Arbeits- und Spanneinrichtung;
Figur 2 einen Querschnitt durch ein weiteres Ausführungsbeispiel der Distraktionsvorrichtung gemäss Figur 1;
Figur 3 einen Querschnitt durch eine Distraktionsvorrichtung gemäss den Figuren 1 und 2 mit einer Zugfeder als Spannelement;
Figur 4 einen Querschnitt durch eine Distraktionsvorrichtung gemäss der Figur 3 mit einer Druckfeder als Arbeitseinrichtung;
Figur 5 einen Querschnitt durch eine Distraktionsvorrichtung gemäss der Figur 4 mit einem Druckfederelement aus Formgedächtnismaterial als Arbeitseinrichtung und einer Druckfeder als Spanneinrichtung.

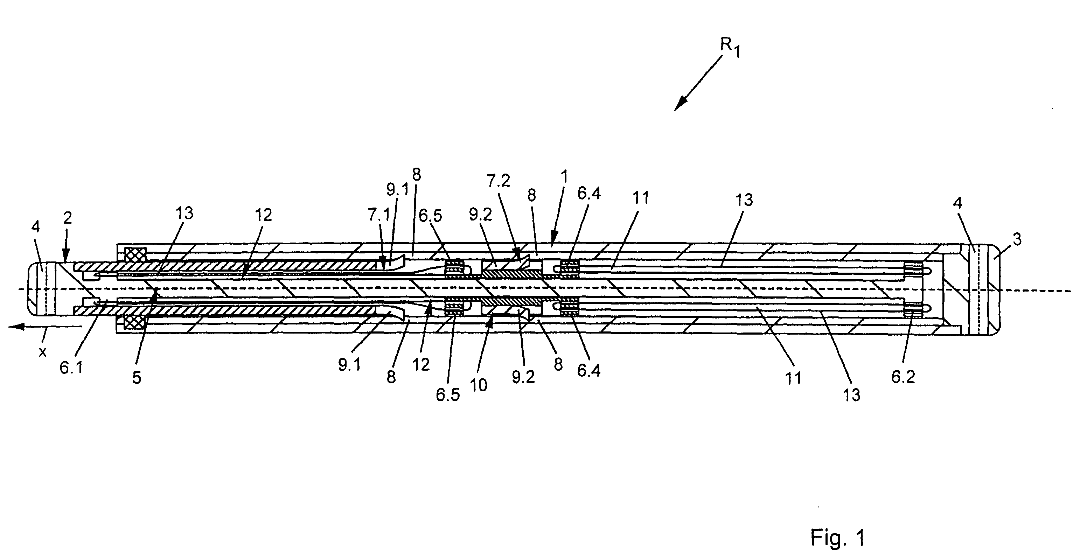

Gemäss Figur 1 weist eine Distraktionsvorrichtung R₁ ein rohrartiges Element 1 auf, in welchem ein entsprechend passendes Element 2 radial geführt und in einer Richtung X axial beweglich ist. Endseitig des Elementes 1 ist ein Deckel 3 als Verschluss vorgesehen. In dem Deckel 3 ist eine Öffnung 4 vorgesehen, um das Element 1 in einem Knochen bspw. mittels eines Schraubenelementes od. dgl. festzulegen bzw. dort zu verankern.

In das Element 2 ist endseits ein Schubelement 5 eingeführt, welches an einem herausragenden Ende ebenfalls mit einer Öffnung 4 zum Festlegen in einem Knochen versehen ist. Das Schubelement 5 ist bevorzugt fest mit dem Element 2 verbunden.

Das Schubelement 5 ist mit einer Aufnahme 6.1 versehen, welche bevorzugt nahe der Öffnung 4 angeordnet ist. Ebenfalls weist das Schubelement 5 andernends, nahe des Deckels 3 im Element 1 eine Aufnahme 6.2 auf.

Bevorzugt endseits nach innen gerichtet ist das Element 2 mit einer Sperreinrichtung 7.1 versehen, welche in hier nicht näher dargestellte Ausrastungen 8 mittels einem Sperrelement 9.1 in Verbindung steht. Die Sperreinrichtung 7.1, insbesondere die Sperrelemente 9.1 sind derart ausgebildet, dass sie in die Ausrastungen 8 des Elementes 1 eingreifen und nur eine Bewegung des Elementes 2 in einer Richtung X und/oder eine Bewegung des Elementes 1 entgegen der Richtung X möglich ist. Bevorzugt ist das Schubelement 5 länger als das Element 2 ausgebildet.

Entsprechend dem Ausführungsbeispiel der vorliegenden Erfindung ist ein Halteelement 10 innerhalb des Elementes 1 angeordnet, welches ebenfalls mit einer Sperreinrichtung 7.2 versehen ist. Das Halteelement 10 wird auf dem Schubelement 5 axial geführt. Diese Sperreinrichtung 7.2 weist ebenfalls Sperrelemente 9.2 auf, welche in entsprechende Ausrastungen 8, die innerhalb des Elementes 1 vorgesehen sind, im Eingriff stehen. Das Halteelement 10 lässt sich innerhalb des Elementes 1 axial hin und her bewegen. Bei einer Bewegung des Halteelementes 10 in Richtung des Deckels 3 nehmen die in die Ausrastungen 8 des Elementes 2 eingreifenden Sperrelemente 9.2 das Element 1 in axialer Richtung entgegen einer Richtung X mit. Die Sperrklinken 9.1 ratschen in nicht sperrender Weise über die Ausrastungen 8. Bei Rückstellung des Halteelementes 10 in die entgegengesetzte Richtung wird das Element 2 durch die in die Ausrastungen 8 eingreifenden Sperrelemente 9.1 des stehenden Elementes 2 an einer axialen Rückwärtsbewegung gehindert und die Sperrelemente 9.2 ratschen in nicht sperrender Weise über die Ausrastungen 8. Das Halteelement 10 ist beidseits mit Aufnahmen 6.4 und 6.5 versehen.

Bei der vorliegenden Erfindung ist eine Arbeitseinrichtung 11 zwischen Halteelement 10 und Schubelement 5, insbesondere zwischen deren Aufnahmen 6.2 und 6.4 eingesetzt. Ferner ist zwischen dem Element 2, insbesondere der Aufnahme 6.1 und dem Halteelement 10, insbesondere dessen Aufnahme 6.5 eine Spanneinrichtung 12 eingesetzt und verbindet das Halteelement 10 mit dem Element 2.

Im vorliegenden Ausführungsbeispiel ist die Arbeitseinrichtung 11 aus einem Draht 13 gebildet, welcher um die Aufnahmen 6.2 und 6.4 aufgewickelt ist. Bevorzugt ist der Draht 13 als Formgedächtnismetall, auch Memory-Metall genannt, ausgebildet. Die Drähte 13 sind ohne elektrischen Kontakt gewickelt bzw. mit einer Isolierung versehen und können von ausserhalb, mit Strom gespeist werden.

Die Spanneinrichtung 12 besteht im vorliegenden Ausführungsbeispiel aus dem Draht 13 aus Memory-Metall. Dieser ist bevorzugt um die Aufnahme 6.1 des Elementes 2 und Aufnahme 6.5 des Halteelementes 10 aufgewickelt.

Diese Distraktonsvorrichtung R₁ wird in einen zu verlängernden Knochen zur Knochenüberbrückung oder Knochenverlängerung in bekannter Weise eingeführt. Die beiden Elemente 1, 2 werden mit den entsprechenden hier nicht dargestellten Knochenteilen fest verbunden, in dem bspw. Befestigungselemente durch die Öffnungen 4 der Elemente 1, 2 eingreifen.

Zur Distraktion wird die Arbeitseinrichtung 11 in Betrieb gesetzt. Dabei wird der Draht 13 oder eine Mehrzahl davon mit Strom beaufschlagt, so dass sich diese auf Grund eines niedrigen Querschnittes und eines hohen ohmschen Widerstandes erwärmen. Diese Erwärmung führt dazu, dass die aus Memory-Metall hergestellten Drähte sich oberhalb einer definierten Temperatur der sogenannten Grenztemperatur zusammenziehen. Je grösser der Abstand zwischen der Aufnahme 6.2 und 6.4 von Schubelement 5 und Halteelement 10 ist, desto grösser wird die Verkürzung der Drähte 13 beim Erwärmen die Arbeitseinrichtung 11. Diese Verkürzung bewirkt, dass das Schubelement 5 druckbeaufschlagt über die Aufnahmen 6.2, 6.4 das Element 2 in Richtung X aus dem Element 1 herausbewegt. Dabei stützt sich das Halteelement mittels der Sperreinrichtung 7.2 an dem Element 1 ab, so dass eine Rückbewegung unterbleibt. Bevorzugt greifen dann die Sperrelemente 9.2 in Ausrastungen 8 des Elementes 1 ein.

Auf diese Weise kann über die Auswahl des Drahtes 13, über den Abstand der Aufnahmen 6.2 und 6.4 Einfluss auf die Distraktion genommen werden. Entsprechend können auch hier die Distraktionskräfte durch die Anzahl der Drähte exakt berechnet und bestimmt werden.

Anschliessend wird die Stromzufuhr unterbrochen und die Arbeitseinrichtung 11 kühlt ab. Die Drähte 13 entspannen sich.

Anschliessend wird die Spanneinrichtung 12 bspw. durch Bestromung in Betrieb gesetzt, so dass sich dessen Drähte 13, welche zwischen der Aufnahme 6.1 und 6.5 des Halteelementes 10 angeordnet sind, erwärmen und in oben beschriebener Weise, da sie als Memory-Metall ausgebildet sind, verkürzen. Diese Verkürzung bewirkt eine Rückstellung des Halteelementes 10, mit oben beschriebener Bewegung.

Gleichzeitig werden die Drähte 13 der Arbeitseinrichtung 11 wieder gespannt.

Dieser Vorgang lässt sich beliebig wiederholen. Auf diese Weise lässt sich das Element 2 aus dem Element 1 und/oder das Element 1 aus dem Element 2 herausfahren. Im Rahmen der vorliegenden Erfindung soll jedoch auch liegen, die Elemente 1, 2 als Vollprofile, Rechteckprofile, od. dgl. auszugestalten.

Ferner soll auch im Rahmen der vorliegenden Erfindung liegen, dass die Elemente 1, 2 gegeneinander nur axial und nicht radial bewegt werden können. Dies kann bspw. mittels Führungsnuten od. dgl. geschehen.

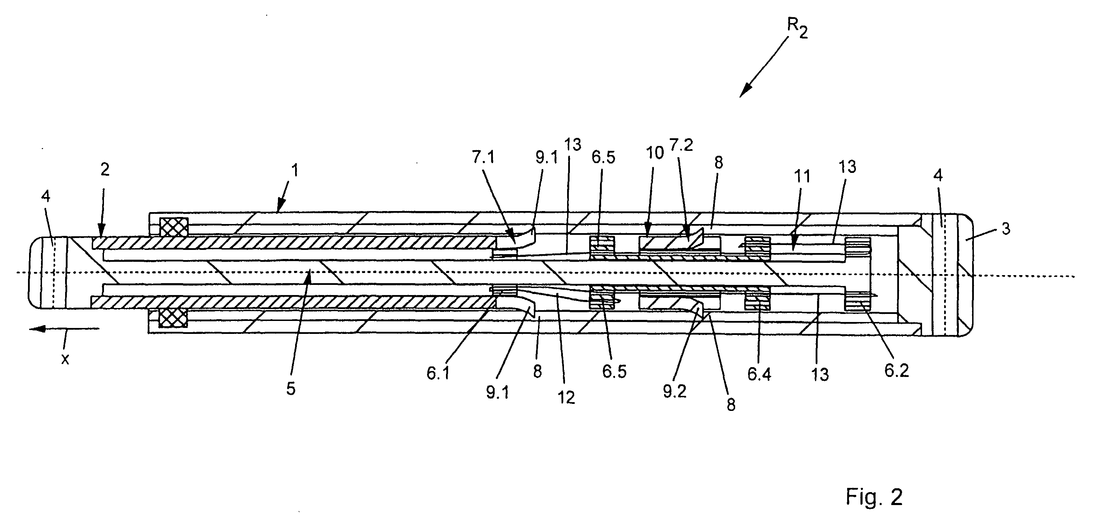

In einem weiteren Ausführungsbeispiel der vorliegenden Erfindung gemäss Figur 2 ist eine Distraktionsvorrichtung R₂ dargstellt, welche im wesentlichen entsprechend der Distraktionsvorrichtung R₁ gemäss Figur 1 aufgebaut ist. Durch eine Umlenkung der Drähte 13 der Arbeitseinrichtung 11 und der Spanneinrichtung 12 wird eine besonders kurze Bauweise erreicht. Dies ist bspw. für sehr kleine und kurze Röhrenknochen im Handbereich von Vorteil.

Daher ist die Aufnahme 6.5 am Element 2 im Bereich der Sperreinrichtung 7.1 angeordnet. Dies hat ferner zum Vorteil, dass sehr kleine Distraktionsschritte vorgenommen werden können und die Distraktionsvorrichtung R₂ insgesamt sehr klein ausgebildet werden kann. Dies ist bspw. für sehr kleine und kurze Knochen von Vorteil.

Die Funktionsweise dieser Distraktionsvorrichtung R₂ entspricht der oben beschriebenen.

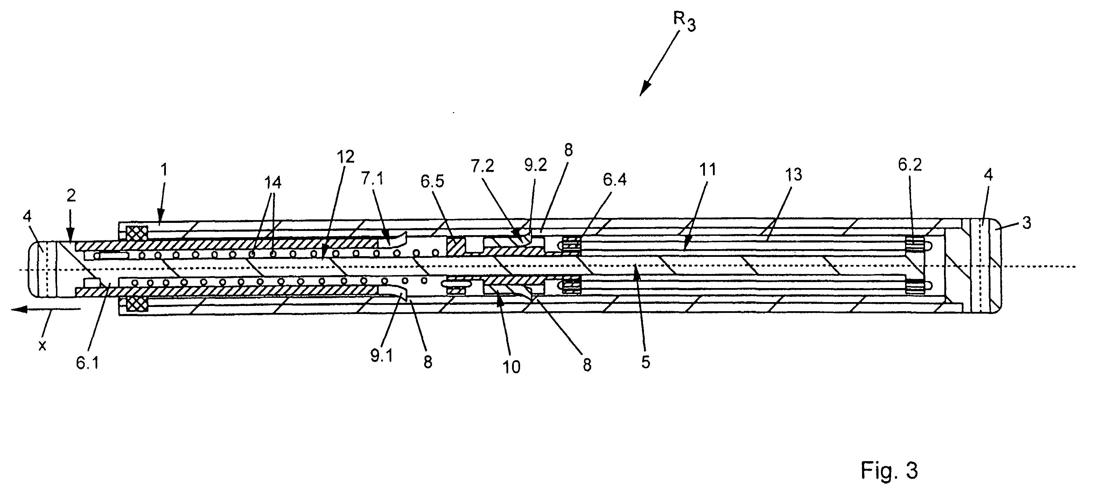

In einem weiteren Ausführungsbeispiel der vorliegenden Erfindung gemäss Figur 3 ist eine Distraktionsvorrichtung R₃ dargstellt, welche in etwa der Distraktionsvorrichtung R₁ gemäss Figur 1 entspricht. Lediglich ist die Spanneinrichtung 12 als Zugfederelement 14 ausgebildet. Die Drähte 13 können hier entfallen. Das Zugfederelement 14 ist einerseits an der Aufnahme 6.1 des Elementes 2 und andererseits an der Aufnahme 6.5 des Halteelementes 10 festgelegt.

Wird die Arbeitseinrichtung 11 in Betrieb gesetzt, so wird nach Erwärmung der Drähte 13 das Schubelement 5 gegenüber dem festgelegten und eingerasteten Halteelementen 10 in Richtung X bewegt und gleichzeitig das Zugfederelement 14 auf Zug gespannt. In oben beschriebener Weise nehmen die Sperrelemente 9.2 das Element 1 in axialer Richtung mit.

Nach dem Unterbrechen der Stromzufuhr kann die Arbeitseinrichtung 11 abkühlen. Das gespannte Zugfederelement 14 dehnt die Drähte 13 bis zu ihrer ursprünglichen Länge. Die Funktionsweise der Sperrelemente bei der Rückstellung ist die gleiche wie bei der Distraktionsvorrichtung R₁. Auf diese Weise kann das Element 2 gegenüber dem Element 1 ausgefahren werden.

Im Rahmen der vorliegenden Erfindung soll jedoch auch liegen, dass bspw. die Elemente 1, 2 als runde oder mehreckartige, rechteckartige Hohlprofile ausgebildet sein können und ineinander verschiebbar sind. Die Elemente 1, 2 können jedoch auch als Vollprofile gegeneinander verschiebbar ausgebildet sein.

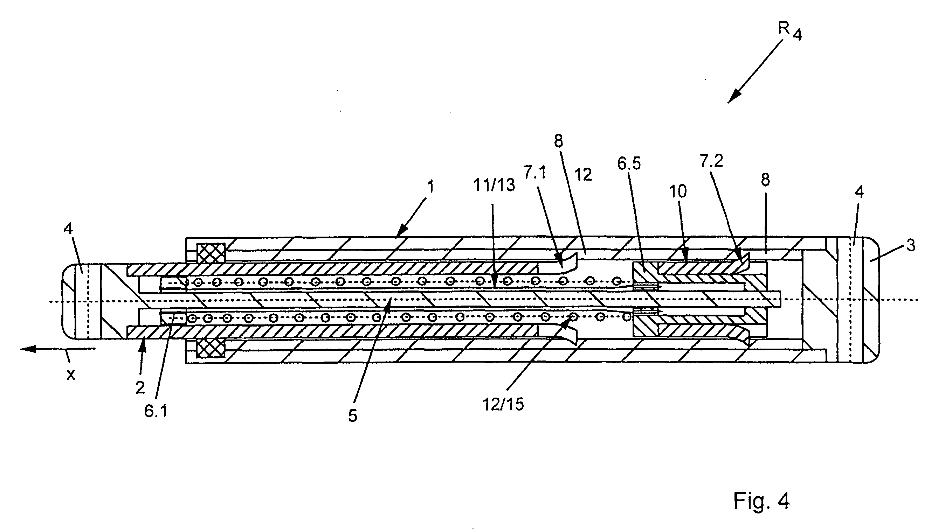

In einem weiteren Ausführungsbeispiel, welches nicht unter den Schutz des Anspruchs 1 fällt, gemäss Figur 4 ist eine Distraktionsvorrichtung R₄ aufgezeigt, bei welcher zwischen dem Halteelement 10, insbesondere dessen Aufnahme 6.5 und der Aufnahme 6.1 des Elementes 2 die Arbeitseinrichtung 11 und die Spanneinrichtung 12 eingesetzt sind. Daher wird eine sehr kurze axiale Bauweise ermöglicht. Bei Erwärmung über eine Grenztemperatur spannt sich der Draht 13 der Spanneinrichtung 12 und spannt ein Druckfederelement 15 der Arbeitseinrichtung 11. Dabei wird das Halteelement 10 in der dort dargestellten Richtung X mitgenommen. Dies erlaubt den bei Figur 1 beschriebenen Ratschenmechanismus.

Nach dem Abkühlen kann sich das Druckfederelement 15⁻ entspannen und nimmt das Element 1 und/oder das Element 2 entgegen oder in der dort dargestellten Richtung X axial mit. Dabei werden gleichzeitig die Drähte 13 auf ihre ursprüngliche Länge gedehnt.

Das Schubelement 5 ist bei diesem Ausführungsbeispiel axial kraftfrei und dient nur zur Führung des Halteelementes 10.

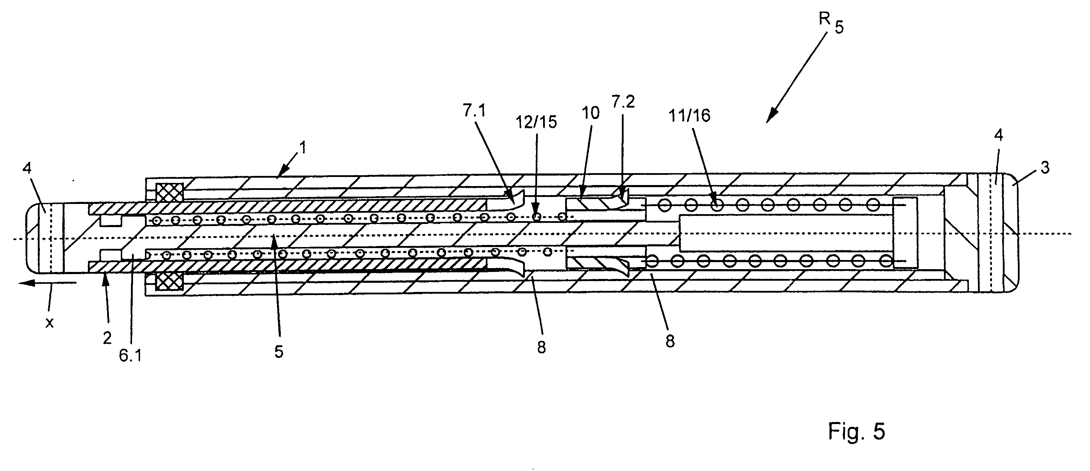

In dem Ausführungsbeispiel gemäss Figur 5, welches ebenfalls nicht unter den Schutzbereich des Anspruchs 1 fällt, ist eine Distraktionsvorrichtung R₅ aufgezeigt, bei welcher die Arbeitseinrichtung 11 als Druckfeder 16 anstelle des Drahtes 13 aus Formgedächtnismetall zu einer Feder gewunden ist, welche bei Überschreiten einer bestimmten Grenztemperatur verlängert wird. Dabei wird ein Druckfederelement 15 der Arbeitseinrichtung 12 gespannt. Die Kräfte können im abgekühlten Zustand der Druckfeder 16 ein Zusammenpressen der Arbeitseinrichtung 12 auf die ursprüngliche Länge hervorrufen. Die entsprechende Funktionsweise des Ratschenmechanismuses, wie er oben beschrieben ist, bleibt gleich.

Ferner soll vom vorliegenden Erfindungsgedanken umfasst sein, dass die Drähte 13, welche aus Memory-Metall hergestellt sind, mit einer Isolierung versehen sind, die entsprechende Verkürzungen und Dehnungen des Drahtes 13 zulassen. Durch den geringen Querschnitt der Drähte 13 kommt es bei einer Bestromung zur Erwärmung. Diese Wärme wird, da die Drähte 13 aus Memory-Metall gebildet sind, zur Verkürzung der Drähte 13 benutzt. Die Drähte 13 der Arbeitseinrichtung 11 und Spanneinrichtung 12 werden über hier nicht dargestellte Geräte zur Einergieeinspeisung angeschlossen, welcher bspw. im Bereich unter der Haut liegt. Über entsprechende induktive Geber kann dann die elektrische Energie zum Inbetriebsetzen der Distraktionsvorrichtungen R₁ bis R₅ übertragen werden. Auf diese Weise können auch Informationen über das distraktive Verhalten übermittelt werden.

Die Drähte 13 oder andere Elemente der Distraktionsvorrichtung können auch von aussen direkt induktiv erwärmt werden, ohne dass bspw. ein Energiewandler vorgesehen sein muss. Dies kann bspw. mit einem wechselnden elektromagnetischen Feld erzeugt werden, welches von aussen an die Extemitäten angelegt wird. Eine elektronische Steuerung oder Regelung kann entfallen.

Es soll ferner daran gedacht sein, entsprechende Temperatursensoren der Distraktionsvorrichtung R₁ bis R₅, insbesondere der Arbeitseinrichtung 11 und Spanneinrichtung 12 zuzuordnen, um eine Temperatursteuerung und Regelung exakt vornehmen zu können. Gleichzeitig können die Temperatursensoren der Kontrolle dienen, ob die Abkühlung beim Entspannen der Arbeitseinrichtung nach einer Erwärmung erfolgt ist. Erst dann wird die Spanneinrichtung 12 in Betrieb genommen.

Zudem kann über derartige Temperatursenoren eine wechselseitge Bestromung der Arbeitseinrichtung 11 bzw. Spanneinrichtung 12 vorgenommen werden, wobei auch die entsprechenden Schaltvorgänge zur Berechnung der erreichten Distraktionsstrecke aufzählbar sind.

Es kann ferner von Vorteil sein, elektrische bzw. mechanische Kontaktelemente für die jeweilige Endposition des Halteelementes 10 bspw. beim Verkürzen mittels der Arbeitseinrichtung bzw. beim Spannen mittels der Spanneinrichtung 12 vorzusehen. Die entsprechenden Kontaktelemente können zur wechselseitigen Bestromung von Arbeitseinrichtung 11 bzw. Spanneinrichtung 12 sowie zum Aufzählen der Kontakte und zur Berechnung der erreichten Distraktionsstrecke dienen.

Ferner kann eine absolute Wegmessung über ein Längenmesssystem, welches nach bspw. dem physikalischen Prinzip der elektrischen Induktion, der Magnetostriktion der Magnetoresistenz oder der Änderung des elektrischen Widerstandes mit zunehmender Länge der ausgefahrenen Elemente 1, 2 funktioniert.

Zur Bestimmung der Verlängerung eines Knochens bzw. zur Überbrückung eines Knochenspaltes können auch Kraftsensoren zur Überwachung der Distraktionskräfte vorgesehen sein. Hierdurch lässt sich ebenfalls Einfluss auf die Verlängerung eines Knochens nehmen.

| Positionszahlenliste | | | | | |
|---|---|---|---|---|---|
| 1 | Element | 34 | | 67 | |
| 2 | Element | 35 | | 68 | |
| 3 | Deckel | 36 | | 69 | |
| 4 | Öffnung | 37 | | 70 | |
| 5 | Schubelement | 38 | | 71 | |
| 6 | Aufnahme | 39 | | 72 | |
| 7 | Sperreinrichtung | 40 | | 73 | |
| 8 | Ausrastung | 41 | | 74 | |
| 9 | Sperrelement | 42 | | 75 | |
| 10 | Halteelement | 43 | | 76 | |
| 11 | Arbeitseinrichtung | 44 | | 77 | |
| 12 | Spanneinrichtung | 45 | | 78 | |
| 13 | Draht | 46 | | 79 | |
| 14 | Zugfederelement | 47 | | | |
| 15 | Druckfederelement | 48 | | | |
| 16 | Druckfeder | 49 | | | |
| 17 | | 50 | | R₁ | Distraktionsvorrichtung |
| 18 | | 51 | | R₂ | Distraktionsvorrichtung |
| 19 | | 52 | | R₃ | Distraktionsvorrichtung |
| 20 | | 53 | | R₄ | Distraktionsvorrichtung |
| 21 | | 54 | | R₅ | Distraktionsvorrichtung |
| 22 | | 55 | | X | Richtung |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

* Translator's note Presumably "or 14" is a clerical error in the German text and should be omitted, or perhaps the first "14" should be "11".

## Patentansprüche

1. Distraktionsvorrichtung zum Auseinanderbewegen eines ein- oder zweiteiligen, ggf. getrennten Knochens zur Verlängerung oder Überbrückung eines Knochenspaltes, umfassend einen ggf. in einen Markraum eines Knochens einführbaren Marknagel, welcher ein erstes Element (1) und ein zweites Element (2) aufweist, welche axial gegeneinander bewegbar sind,
**dadurch gekennzeichnet,**
**dass** zum axialen Auseinanderbewegen des ersten Elementes (1) gegenüber dem zweiten Element (2) ein Halteelement (10) vorgesehen ist, welches gegenüber dem ersten Element (1) abgestützt ist und einends zwischen dem Halteelement (10) und dem zweiten Element (2) einerseits eine Arbeitseinrichtung (11) bestehend aus zumindest einem Draht oder einem Rohr- oder rohrartigem Element aus einer Formgedächtnis-Legierung und andernends zwischen Halteelement (10) und dem zweiten Element (2) andererseits eine Spanneinrichtung (12) vorgesehen ist, wobei eine Distraktion des ersten Elementes (1) gegenüber dem zweiten Element (2) durch Verkürzen der Arbeitseinrichtung (11) erfolgt und ein Schubelement (5) des zweiten Elementes (2) gegenüber dem an dem ersten Element (1) fixierten Halteelement (10) in Distraktions-Richtung (X) bewegbar ist.

2. Distraktionsvorrichtung nach Anspruch 1, **dadurch gekennzeichnet, dass** an das bewegbare Element (1 oder 2) ein Schubelement (5) anschliesst, welches das Halteelement (10) durchgreift.

3. Distraktionsvorrichtung nach Anspruch 2, **dadurch gekennzeichnet, dass** das Schubelement (5) einends mit einer Aufnahme (6.2) versehen ist.

4. Distraktionsvorrichtung nach Anspruch 3, **dadurch gekennzeichnet, dass** die Arbeitseinrichtung (11) zwischen Aufnahme (6.2) des Schubelementes (5) und Halteelement (10) angeordnet und mit diesen, ggf. an dessen Aufnahme (6.4 oder 6.5), verbunden ist.

5. Distraktionsvorrichtung nach wenigestens einem der Ansprüche 1 bis 4, **dadurch gekennzeichnet, dass** dem Halteelement (10) und/oder dem zweiten Element (2) oder dem ersten Element (1) wenigstens eine Sperreinrichtung (7.1, 7.2), insbesondere als Ausrastungen (8) mit darin eingreifenden Sperrelementen (9.1, 9.2) zugeordnet ist, welche durch Kraftübertragung des Halteelementes (10) auf dass Element (2) eine Rückbewegung des Elementes (1) entgegen einer Richtung (X) verhindert.

6. Distraktionsvorrichtung nach Anspruch 5, **dadurch gekennzeichnet, dass** das Sperrelement (7.1) rastbar eine Rückbewegung des zweiten Elementes (2) gegenüber dem ersten Element (1) verhindert.

7. Distraktionsvorrichtung nach wenigstens einem der Ansprüche 1 bis 6, **dadurch gekennzeichnet, dass** die Elemente (1, 2) als Hohlprofile ausgebildet sind, wobei das zweiten Element (2) innerhalb des ersten Elementes (1) verschiebbar angeordnet ist.

8. Distraktionsvorrichtung nach wenigstens einem der Ansprüche 1 bis 7, **dadurch gekennzeichnet, dass** die Arbeitsseinrichtung (11) zumindest teilweise aus Formgedächtnismaterial hergestellt ist.

9. Distraktionsvorrichtung nach wenigstens einem der Ansprüche 1 bis 8, **dadurch gekennzeichnet, dass** die Arbeitseinrichtung (11) aus einer Mehrzahl von Drähten (13) gebildet ist, welche zueinander isoliert sind, wobei die Drähte (13) aus Formgedächtnismaterial hergestellt sind.

10. Distraktionsvorrichtung nach wenigstens einem der Ansprüche 1 bis 9, **dadurch gekennzeichnet, dass** durch Betätigen der Arbeitseinrichtung (11) das Schubelement (5) des zweiten Elementes (2) gegen das Halteelement (10) bewegbar ist.

11. Distraktionsvorrichtung nach Anspruch 9 oder 10, **dadurch gekennzeichnet, dass** bei Bestromung der Arbeitseinrichtung (11), insbesondere der Drähte (13) eine Erwärmung erfolgt, welche eine Verkürzung der Arbeitseinrichtung (11) bewirkt.

12. Distraktionsvorrichtung nach Anspruch 11, **dadurch gekennzeichnet, dass** nach dem Betreiben der Arbeitseinrichtung (11) und nach ggf. Unterbrechung einer Bestromung ein Abkühlen und unter Krafteinwirkung der Spanneinrichtung (12) eine Verformung der Elemente der Arbeitseinrichtung (11) in deren ursprüngliche Form erfolgt.

13. Distraktionsvorrichtung nach wenigstens einem der Ansprüche 1 bis 12, **dadurch gekennzeichnet, dass** die Spanneinrichtung (12) zumindest teilweise aus Formgedächtnismaterial hergestellt und zwischen einer Aufnahme (6.1) des Elementes (2) und Aufnahme (6.2 und/oder 6.5) vorgesehen ist.

14. Distraktionsvorrichtung nach wenigstens einem der Ansprüche 1 bis 13, **dadurch gekennzeichnet, dass** die Spanneinrichtung (12) aus einem Zugfederelement (14) oder einem Druckfederelement (15) und/oder aus einer Mehrzahl von Drähten (13) gebildet ist, welche zueinander isoliert sind, wobei die Drähte (13) aus Formgedächtnismaterial hergestellt sind.

15. Distraktionsvorrichtung nach Anspruch 14 oder 14, **dadurch gekennzeichnet, dass** bei Bestromung der Spanneinrichtung (12), insbesondere der Drähte (13) eine Erwärmung erfolgt, welche eine Verkürzung der Spanneinrichtung (12) bewirkt.

16. Distraktionsvorrichtung nach Anspruch 15, **dadurch gekennzeichnet, dass** durch Verkürzung der Spanneinrichtung (12), insbesondere der Drähte (13) das Halteelement (10) gegenüber dem zweiten Element (2) in Distraktions-Richtung (X), ggf. rastbar bewegbar ist.

17. Distraktionsvorrichtung nach Anspruch 16, **dadurch gekennzeichnet, dass** nach dem Betreiben der Spanneinrichtung (12) ein Abkühlen durch Unterbrechung der Bestromung und damit eine Dehnung der Spanneinrichtung (12), insbesondere der Drähte (13) erfolgt.

18. Distraktionsvorrichtung nach wenigstens einem der Ansprüche 1 bis 17, **dadurch gekennzeichnet, dass** Arbeitseinrichtung (11) und Spanneinrichtung (12) alternierend betrieben sind.

19. Distraktionsvorrichtung nach wenigestens einem der Ansprüche 1 bis 18, **dadurch gekennzeichnet, dass** der Arbeitseinrichtung (11) oder der Spanneinrichtung (12) zumindest eine Heizeinrichtung zugeordnet ist.

20. Distraktionsvorrichtung nach wenigstens einem der Ansprüche 1 bis 19, **dadurch gekennzeichnet, dass** die Arbeitseinrichtung (11) und/oder Spanneinrichtung (12) als Rohr- oder rohrartige Elemente aus Formgedächtnismetall gebildet sind, welchen zur Verkürzung eine Heizeinrichtung zugeordnet ist.

21. Distraktionsvorrichtung nach wenigstens einem der Ansprüche 1 bis 20, **dadurch gekennzeichnet, dass** eine Versorgung einer Arbeitseinrichtung (11) und/oder Spanneinrichtung (12) mit Energie berührungslos ggf. über Induktion erfolgt.

22. Distraktionsvorrichtung nach wenigstens einem der Ansprüche 1 bis 21, **dadurch gekennzeichnet, dass** der Arbeitseinrichtung (11) und/oder der Spanneinrichtung (12) Temperaturmesselemente, insbesondere Temperatursenoren zur Überwachung, Steuerung und Regelung der Temperaturen beim Erwärmen und Abkühlen zugeordnet sind.

23. Distraktionsvorrichtung nach wenigstens einem der Ansprüche 1 bis 22, **dadurch gekennzeichnet, dass** der Arbeitseinrichtung (11) und/oder der Spanneinrichtung (12) Sensorelemente und/oder Kontrollelemente zugeordnet sind, welche die Anzahl der Schaltvorgänge ermitteln und eine Distraktionsstrecke bestimmen.

24. Distraktionsvorrichtung nach wenigestens einem der Ansprüche 1 bis 23, **dadurch gekennzeichnet, dass** dem ersten und zweiten Element (1, 2), Kontaktelemente zum Erkennen von Endlagen des Halteelementes (10) und/oder des ersten Elementes (1) zugeordnet sind.

25. Distraktionsvorrichtung nach wenigstens einem der Ansprüche 1 bis 24, **dadurch gekennzeichnet, dass** Kraftsensoren zur Überwachung der Distraktionskräfte dem ersten und zweiten Element (1, 2), und/oder der Spanneinrichtung (12) und/oder Arbeitseinrichtung (11) zugeordnet sind.

26. Distraktionsvorrichtung nach wenigstens einem der Ansprüche 1 bis 25, **dadurch gekennzeichnet, dass** das erste und zweite Element (1, 2) verdrehgesichert, ggf. über Führungsnuten, Führungsnasen od. dgl. axial ineinander oder aneinander 20 geführt, bewegbar angeordnet ist.

27. Distraktionsvorrichtung nach wenigstens einem der Ansprüche 9 bis 26, **dadurch gekennzeichnet, dass** der zumindest eine Draht (13) der Arbeitseinrichtung (11) und/oder der Spanneinrichtung (12) im Halteelement (10) und/oder in der zumindest einen Aufnahme (6.1 bis 6.5) durchgeführt ist.

28. Distraktionsvorrichtung nach wenigstens einem der Ansprüche 1 bis 27, **dadurch gekennzeichnet, dass** Arbeitseinrichtung (11) und/oder Spanneinrichtung (12) von extern, ggf. induktiv erwärmbar sind.

## Claims

1. Distraction apparatus for moving apart a one- or two-part, possibly divided bone to extend or bridge a bone gap, said apparatus including a medullary nail, which is possibly insertable into a medullary chamber of a bone and has a first element (1) and a second element (2) which are axially displaceable relative to each other, **characterised in that** a retaining element (10) is provided for axially moving apart the first element (1) relative to the second element (2) and is supported relative to the first element (1), and on the one hand an operating means (11), comprising at least one wire or one tube element or tubular element and formed from a shape-memorising alloy, is provided at one end between the retaining element (10) and the second element (2) and, on the other hand, a tensioning means (12) is provided at the other end between retaining element (10) and the second element (2), a distraction of the first element (1) relative to the second element (2) being effected by shortening the operating means (11), and a pushing element (5) of the second element (2) being displaceable relative to the retaining element (10), which is secured on the first element (1), in the distraction direction (X).

2. Distraction apparatus according to claim 1, **characterised in that** a pushing element (5), which extends through the retaining element (10), communicates with the displaceable element (1 or 2).

3. Distraction apparatus according to claim 2, **characterised in that** the pushing element (5) is provided with a receiver (6.2) at one end.

4. Distraction apparatus according to claim 3, **characterised in that** the operating means (11) is disposed between receiver (6.2) of the pushing element (5) and retaining element (10) and is connected thereto, possibly on the receiver (6.4 or 6.5) thereof.

5. Distraction apparatus according to at least one of claims 1 to 4, **characterised in that** at least one blocking means (7.1, 7.2), more especially in the form of unlocking devices (8) with blocking elements (9.1, 9.2) engaging therein, is associated with the retaining element (10) and/or the second element (2) or the first element (1), which blocking means prevents a rearward movement of the element (1) in opposition to a direction (X) by a transfer of force from the retaining element (10) to the element (2).

6. Distraction apparatus according to claim 5, **characterised in that** the blocking element (7.1) prevents a rearward movement of the second element (2) relative to the first element (1) in a lockable manner.

7. Distraction apparatus according to at least one of claims 1 to 6, **characterised in that** the elements (1, 2) are in the form of hollow profiles, the second element (2) being displaceably disposed within the first element (1).

8. Distraction apparatus according to at least one of claims 1 to 7, **characterised in that** the operating means (11) is produced at least partially from shape-memorising material.

9. Distraction apparatus according to at least one of claims 1 to 8, **characterised in that** the operating means (11) is formed from a plurality of wires (13) which are insulated from one another, the wires (13) being produced from shape-memorising material.

10. Distraction apparatus according to at least one of claims 1 to 9, **characterised in that** the pushing element (5) of the second element (2) is movable towards the retaining element (10) by actuating the operating means (11).

11. Distraction apparatus according to claim 9 or 10, **characterised in that**, by supplying the operating means (11), more especially the wires (13), with current, a heating occurs, which causes a shortening of the operating means (11).

12. Distraction apparatus according to claim 11, **characterised in that**, after the operation of the operating means (11) and after possibly interrupting a current supply, a cooling occurs and, by the effect of the force of the tensioning means (12), a deformation of the elements of the operating means (11) into their original shape occurs.

13. Distraction apparatus according to at least one of claims 1 to 12, **characterised in that** the tensioning means (12) is produced at least partially from shape-memorising material and is provided between one receiver (6.1) of the element (2) and receiver (6.2 and/or 6.5).

14. Distraction apparatus according to at least one of claims 1 to 13, **characterised in that** the tensioning means (12) is formed from a tension spring element (14) or a compression spring element (15) and/or from a plurality of wires (13), which are insulated from one another, the wires (13) being produced from shape-memorising material.

15. Distraction apparatus according to claim 14 or 14*, **characterised in that**, by supplying the tensioning means (12), more especially the wires (13), with current, a heating occurs, which causes a shortening of the tensioning means (12).

16. Distraction apparatus according to claim 15, **characterised in that**, by shortening the tensioning means (12), more especially the wires (13), the retaining element (10) is movable, possibly in a lockable manner, relative to the second element (2) in the distraction direction (X).

17. Distraction apparatus according to claim 16, **characterised in that**, after the operation of the tensioning means (12), a cooling is effected by interrupting the current supply and, in consequence, an expansion of the tensioning means (12), more especially of the wires (13), is effected.

18. Distraction apparatus according to at least one of claims 1 to 17, **characterised in that** the operating means (11) and tensioning means (12) are operated alternately.

19. Distraction apparatus according to at least one of claims 1 to 18, **characterised in that** at least one heating means is associated with the operating means (11) or the tensioning means (12).

20. Distraction apparatus according to at least one of claims 1 to 19, **characterised in that** the operating means (11) and/or tensioning means (12) are/is in the form of tube elements or tubular elements formed from shape-memorising metal, with which a heating means is associated for shortening purposes.

21. Distraction apparatus according to at least one of claims 1 to 20, **characterised in that** an operating means (11) and/or tensioning means (12) is supplied with power in a contactless manner, possibly via induction.

22. Distraction apparatus according to at least one of claims 1 to 21, **characterised in that** temperature measuring elements, more especially temperature sensors for monitoring, controlling and regulating the temperatures during heating and cooling, are associated with the operating means (11) and/or the tensioning means (12).

23. Distraction apparatus according to at least one of claims 1 to 22, **characterised in that** sensor elements and/or checking elements, which ascertain the number of switching procedures and determine a distraction path, are associated with the operating means (11) and/or the tensioning means (12).

24. Distraction apparatus according to at least one of claims 1 to 23, **characterised in that** contact elements for detecting end positions of the retaining element (10) and/or of the first element (1) are associated with the first and second elements (1, 2).

25. Distraction apparatus according to at least one of claims 1 to 24, **characterised in that** force sensors for monitoring the distraction forces are associated with the first and second elements (1, 2) and/or the tensioning means (12) and or operating means (11).

26. Distraction apparatus according to at least one of claims 1 to 25, **characterised in that** the first and second elements (1, 2) are movably disposed in a twist-preventing manner, possibly guided axially in each other or on each other via guide grooves, guide projections or the like.

27. Distraction apparatus according to at least one of claims 9 to 26, **characterised in that** the at least one wire (13) of the operating means (11) and/or of the tensioning means (12) is traversed in the retaining element (10) and/or in the at least one receiver (6.1 to 6.5).

28. Distraction apparatus according to at least one of claims 1 to 27, **characterised in that** operating means (11) and/or tensioning means (12) are heatable from externally, possibly inductively.

## Revendications

1. Dispositif de distraction d'os pour l'écartement d'un os d'une pièce ou de deux pièces éventuellement séparées, destiné à allonger ou à surplomber une fente d'os, comprenant une broche, pouvant éventuellement être introduite dans un espace de moelle, qui présente un premier élément (1) et un deuxième élément (2) qui sont mobiles axialement l'un par rapport à l'autre,
**caractérisé par le fait**
**qu'**il est prévu, pour l'écartement axial du premier élément (1) par rapport au deuxième élément (2), un élément de retenue (10) qui est supporté par rapport au premier élément (1) et qu'il est prévu, à une extrémité, entre l'élément de retenue (10) et le deuxième élément (2), d'une part, un dispositif de travail (11), composé d'au moins un fil ou d'un élément en forme de tube ou tubulaire, réalisé en un alliage à mémoire de forme et, à l'autre extrémité, entre l'élément de retenue (10) et le deuxième élément (2), d'autre part, un dispositif de serrage (12), une distraction du premier élément (1) par rapport au deuxième élément (2) ayant lieu par raccourcissement du dispositif de travail (11) et un élément coulissant (5) du deuxième élément (2) étant déplaçable dans le sens de la distraction (X) par rapport à l'élément de retenue (10) fixé au premier élément (1).

2. Dispositif de distraction selon la revendication 1, **caractérisé par le fait qu'**à l'élément mobile (1 ou 2) vient se raccorder un élément coulissant (5) qui traverse l'élément de retenue (10).

3. Dispositif de distraction selon la revendication 2, **caractérisé par le fait que** l'élément coulissant (5) est pourvu, à une extrémité, d'un dispositif de réception (6.2).

4. Dispositif de distraction selon la revendication 3, **caractérisé par le fait que** le dispositif de travail (11) est disposé entre le dispositif de réception (6.2) de l'élément coulissant (5) et l'élément de retenue (10) et est connecté à ce dernier, éventuellement à son dispositif de réception (6.4 ou 6.5).

5. Dispositif de distraction selon au moins l'une des revendications 1 à 4, **caractérisé par le fait qu'**à l'élément de retenue (10) et/ou au deuxième élément (2) ou au premier élément (1) est associé au moins un dispositif de blocage (7.1, 7.2), en particulier sous forme de trous d'encliquetage (8), avec des éléments de blocage (9.1, 9.2) s'introduisant dans ces derniers, qui, par transmission de force de l'élément de retenue (10) sur l'élément (2), empêche un recul de l'élément (1) à l'encontre d'une direction (X).

6. Dispositif de distraction selon la revendication 5, **caractérisé par le fait que** l'élément de blocage (7.1) empêche, par encliquetage, un recul du deuxième élément (2) par rapport au premier élément (1).

7. Dispositif de distraction selon au moins l'une des revendications 1 à 6, **caractérisé par le fait que** les éléments (1, 2) sont réalisés sous forme de profilés creux, le deuxième élément étant disposé de manière déplaçable à l'intérieur du premier élément (1).

8. Dispositif de distraction selon au moins l'une des revendications 1 à 7, **caractérisé par le fait que** le dispositif de travail (11) est réalisé au moins partiellement en un matériau à mémoire de forme.

9. Dispositif de distraction selon au moins l'une des revendications 1 à 8, **caractérisé par le fait que** le dispositif de travail (11) est réalisé en une pluralité de fils (13) qui sont isolés l'un de l'autre, les fils (13) étant réalisés en un matériau à mémoire de forme.

10. Dispositif de distraction selon au moins l'une des revendications 1 à 9, **caractérisé par le fait que** l'élément coulissant (5) du deuxième élément (2) peut être déplacé par rapport à l'élément de retenue (10) en actionnant le dispositif de travail (11).

11. Dispositif de distraction selon la revendication 9 ou 10, **caractérisé par le fait que** lors d'une alimentation de courant vers le dispositif de travail (11), en particulier les fils (13), a lieu un échauffement qui provoque un raccourcissement du dispositif de travail (11).

12. Dispositif de distraction selon la revendication 11, **caractérisé par le fait qu'**après le fonctionnement du dispositif de travail (11) et éventuellement après interruption de l'alimentation de courant ont lieu un refroidissement et, sous l'influence de la force du dispositif de serrage (12), une déformation des éléments du dispositif de travail (11) à leur forme originale.

13. Dispositif de distraction selon au moins l'une des revendications 1 à 12, **caractérisé par le fait que** le dispositif de serrage (12) est réalisé au moins partiellement en un matériau à mémoire de forme et est prévu entre un dispositif de réception (6.1) de l'élément (2) et le dispositif de réception (6.2 et/ou 6.5).

14. Dispositif de distraction selon au moins l'une des revendications 1 à 13, **caractérisé par le fait que** le dispositif de serrage (12) est constitué d'un élément de ressort de traction (14) ou d'un élément de ressort de poussée (15) et/ou d'une pluralité de fils (13) qui sont isolés l'un de l'autre, les fils (13) étant réalisés en un matériau à mémoire de forme.

15. Dispositif de distraction selon la revendication 14 ou 14, **caractérisé par le fait que** lors de l'alimentation de courant vers le dispositif de serrage (12), en particulier les fils (13), a lieu un échauffement qui provoque un raccourcissement du dispositif de serrage (12).

16. Dispositif de distraction selon la revendication 15, **caractérisé par le fait que** par le raccourcissement du dispositif de serrage (12), en particulier les fils (13), le dispositif de retenue (10) peut se déplacer dans la direction de distraction (X), éventuellement de manière encliquetable, par rapport au deuxième élément (2).

17. Dispositif de distraction selon la revendication 15, **caractérisé par le fait qu'**après le fonctionnement du dispositif de serrage (12) a lieu un refroidissement par interruption de l'alimentation de courant et, de ce fait, une dilatation du dispositif de serrage (12), en particulier les fils (13).

18. Dispositif de distraction selon au moins l'une des revendications 1 à 17, **caractérisé par le fait que** le dispositif de travail (11) et le dispositif de serrage (12) fonctionnent en alternance.

19. Dispositif de distraction selon au moins l'une des revendications 1 à 18, **caractérisé par le fait qu'**au dispositif de travail (11) ou au dispositif de serrage (12) est associé au moins un dispositif chauffant.

20. Dispositif de distraction selon au moins l'une des revendications 1 à 19, **caractérisé par le fait que** le dispositif de travail (11) et/ou le dispositif de serrage (12) sont réalisés sous forme d'éléments en forme de tube ou tubulaires en un métal à mémoire de forme, auxquels est associé, pour le raccourcissement, un dispositif chauffant.

21. Dispositif de distraction selon au moins l'une des revendications 1 à 20, **caractérisé par le fait qu'**une alimentation en énergie d'un dispositif de travail (11) et/ou dispositif de serrage (12) a lieu sans contact, éventuellement par induction.

22. Dispositif de distraction selon au moins l'une des revendications 1 à 21, **caractérisé par le fait qu'**au dispositif de travail (11) et/ou au dispositif de serrage (12) sont associés des éléments de mesure de température, en particulier des capteurs de température, destinés à surveiller, régler et réguler les températures lors de l'échauffement et du refroidissement.

23. Dispositif de distraction selon au moins l'une des revendications 1 à 22, **caractérisé par le fait qu'**au dispositif de travail (11) et/ou au dispositif de serrage (12) sont associés des éléments capteurs et/ou des éléments de contrôle qui déterminent le nombre d'opérations de commutation et déterminent un trajet de distraction.

24. Dispositif de distraction selon au moins l'une des revendications 1 à 23, **caractérisé par le fait qu'**au premier et deuxième élément (1, 2) sont associés des éléments de contact destinés à détecter des fins de course de l'élément de retenue (10) et/ou du premier élément (1).

25. Dispositif de distraction selon au moins l'une des revendications 1 à 24, **caractérisé par le fait que** des capteurs de force destinés à surveiller les forces de distraction sont associés au premier et deuxième élément (1, 2) et/ou au dispositif de serrage (12) et/ou au dispositif de travail (11).

26. Dispositif de distraction selon au moins l'une des revendications 1 à 25, **caractérisé par le fait que** le premier et deuxième élément (1, 2) est disposé de manière déplaçable l'un dans l'autre ou l'un hors de l'autre, protégé contre une torsion, éventuellement guidé par l'intermédiaire de rainures de guidage, de saillies de guidage ou autres.

27. Dispositif de distraction selon au moins l'une des revendications 1 à 26, **caractérisé par le fait que** l'au moins un fil (23) du dispositif de travail (11) et/ou du dispositif de serrage (12) est réalisé dans l'élément de retenue (10) et/ou dans l'au moins un dispositif de réception (6.1 à 6.5).

28. Dispositif de distraction selon au moins l'une des revendications 1 à 27, **caractérisé par le fait que** le dispositif de travail (11) et/ou le dispositif de serrage (12) sont chauffés de l'extérieur, éventuellement de manière inductive.
